# EUROPEAN PATENT APPLICATION

(11) **EP 0 894 864 A1**
(43) Date of publication of application: **03.02.1999**
(21) Application number: 97112983.8
(22) Date of filing: 29.07.1997
(51) Int. Cl.: C12N 15/82, C12N 9/02, C12Q 1/68, A01H 5/00

(54) **Genes encoding enzymes of the ACDH-family in plants and methods for producing transgenic seeds or plants with enhanced content or altered compsition of fatty acids and/or amino acids**

(71) Applicant: Brennicke, Axel, Dr., 89143 Blaubeuren (DE); Binder, Stefan, Dr., 89134 Bermaringen (DE)
(72) Inventor: Brennicke, Axel, Dr., 89143 Blaubeuren (DE); Binder, Stefan, Dr., 89134 Bermaringen (DE)

(57) **Abstract**

The present invention relates to genes encoding enzymes of the ACDH family in plants. The present invention also relates to nucleic acid sequences which are complementary to said genes or parts thereof, for example antisense RNA or DNA sequences or ribozymes, which are capable of lowering or inhibiting the synthesis of said enzyms. The present invention, furthermore, relates to expression vectors comprising inter alia said genes or the nucleic acid sequences which are complementary to said genes.

Moreover, the present invention relates to methods for enhancing the content or altering the composition and/or relative amounts of fatty acids and/or amino acids and compounds containing these such as any lipids, oils, waxes and fats or storage proteins in a seed, plant cell or plant or for inhibiting or postponing germination in a seed using the above nucleic acid sequences and/or other nucleic acid sequences coding for enzymes of the β-oxidative pathway(s). Finally, the present invention relates to transgenic seeds, plant cells or plants which can be obtained by introducing the above nucleic acid sequences.

SEQUENCE LISTING

(1) GENERAL INFORMATION:

(i) APPLICANT:
(A) NAME: BRENNICKE, Axel Dr. / Binder, Stefan Dr.
(B) STREET: Karlstr. 71
(C) CITY: Blaubeuren
(E) COUNTRY: Germany
(F) POSTAL CODE (ZIP): D-89143
(G) TELEPHONE: 49-7344-21385

(ii) TITLE OF INVENTION: Genes encoding enzymes of the acdh-family in
plants and methods for producing transgenic seeds or
plants with enhanced content or altered composition of
fatty acids and/or amino acids

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 1321 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Arabidopsis thaliana
(B) STRAIN: Columbia

(vii) IMMEDIATE SOURCE:
(B) CLONE: ivd

(xi) SEQUENCE DESCRIPTION: SEQ ID NO: 1:
   CACATTCTTC CGATCTTTCT TGTTTTTAAC TTACCGGTAA TAACGAAGAT ATGCAGAGGT 60
   TTTTCTCCGC CAGATCGATT CTCGGTTACG CCGTCAAGAC GCGGAGGAGG TCTTTCTCTT 120
   CTCGTTCTTC GTCTCTCCTA TTCGACGACA CTCAGTTACA GTTTAAAGAA AGTGTATCCA 180
   AGTTTGCGCA AGATAATATC GCTCCTCATG CAGAAAGAAT TGATAAAACT AATTCATTTC 240
   CAAAGGATGT AAACTTATGG AAGCTAATGG GTGAGTTTAA TCTCCATGGA ATCACTGCGC 300
   CAGAGGAATA TGGAGGGCTA GGTCTTGGTT ACTTGTATCA TTGTATTGCA ATGGAGGAAA 360
   TCAGTCGGGC CTCGGGTTCA GTTGCTTTGT CTTATGGTGC TCATTCCAAC CTTTGCATCA 420
   ATCAATTGGT GAGGAATGGA ACTGCAGCCC AAAAGGAAAA GTATTTGCCA AAGCTTATCA 480
   GTGGAGAGCA TGTTGGTGCT CTTGCAATGA GTGAACCTAA TGCTGGTTCA GATGTTGTTG 540
   GCATGAAATG CAAGGCTGAG AAGGTAGATG GTGGTTATAT ACTAAATGGC AACAAGATGT 600
   GGTGCACTAA TGGTCCATCT GCTGAAACAC TGGTTGTTTA CGCGAAAACT GATACAAAAG 660
   CAGGTTCCAA AGGAATCACG GCTTTCATCA TAGAGAAAGG AATGACCGGA TTCAGTACTG 720
   CTCAGAAATT GGACAAACTA GGAATGCGGG GAAGCGATAC GTGTGAGCTT GTTTTTGAGA 780
   ATTGCTTCGT TCCAGAGGAA AATATTCTAG ACAAGGAAGG AAAAGGAGTG TATGTTCTGA 840
   TGTCAGGTCT GGATTTGGAG AGACTTGTTT TAGCAGCTGG GCCCTTAGGG ATCATGCAGG 900
   CATGCCTCGA CAATGTTCTT CCCTATATTC GCCAGAGAGA ACAATTTGGT CGTCCAGTTG 960
   GGGAATTTCA GTTTATACAG GGTAAAGTTG CTGATATGTA CACTGCATTA CAATCTTCAA 1020
   GGTCATACGT TTACTCTGTT GCGAGGGACT GTGACAATGG GAAAGTTGAC CCAAAGGACT 1080
   GCGCCGGAAC TATTCTTTGT GCGGCCGAGA GAGCAACGCA GGTCGCTTTA CAGGCGATAC 1140
   AATGTTTAGG CGGAAATGGA TACATAAACG AATATGCAAC AGGACGCCTT CTAAGAGATG 1200
   CAAAGCTATA TGAAATCGGT GCAGGGACAA GCGAGATCAG AAGGATTGTC ATCGGTCGCG 1260
   AGCTTTTCAA AGAAGAATAG AGATAAGTCT GTTTTCTGCA TTGCCAATGC GACATTTTCA 1320
   T 1321

(2) INFORMATION FOR SEQ ID NO: 2:

(i) SEQUENCE CHARACTERISTICS:
(A) LENGTH: 1496 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Arabidopsis thaliana
(B) STRAIN: Columbia

(vii) IMMEDIATE SOURCE:
(B) CLONE: GCDH

(xi) SEQUENCE DESCRIPTION: SEQ ID NO: 2:

GTGCTTTCAT CTGCAGATCG AGCTAGTAAT GAGAAGAAGG TGAAGAGTTC ATACTTTGAT 60

TTGCCACCTA TGGAAATGTC TGTAGCATTT CCTCAAGCAA CTCCAGCCTC TACATTTCCA 120

CCTTGTACTT CAGACTATTA TCATTTCAAT GATCTACTGA CTCCGGAAGA ACAAGCTATC 180
CGGAAGAAAG TGAGGGAATG CATGGAGAAA GAAGTTGCTC CAATAATCAC AGAGTACTCG 240
GAGAAAGCAG AATTTCCATT CCATATCACT CCAAAGCTTG GGGCTATGGG TGTTGCTGGT 300
GGCTCGATCA AGGGTTATGG ATGTCCTGGT CTCTCCATCA CCGCCAATGC AATTGCCACA 360
GCAGAAATAG CTAGAGTTGA TGCAAGTTGT TCGACTTTCA TTTTGGTGCA TTCTTCTTTG 420
GGCATGCTCA CTATTGCACT CTGTGGATCA GAAGCACAGA ATGAGAAGTA TTTGCCTTCT 480
TTGGCTCAAT TGAATACTGT GGCTTGTTGG GCTTTGACAG AGCCCGACAA TGGAAGCGAT 540
GCAAGTGGTC TAGGAACGAC TGCAACAAAG GTTGAAGGAG GTTGGAAAAT TAATGGACAA 600
AAGCGTTGGA TTGGAAACAG CACCTTTGCA GATCTGTTGA TCATCTTTGC GAGGAATACA 660
ACAACTAACC AAATCAACGG ATTCATAGTC AAGAAAGATG CGCCTGGCCT AAAGGCTACT 720
AAGATCCCAA ATAAAATAGG TTTACGTATG GTTCAAAATG GAGATATTCT ACTACAGAAT 780
GTCTTTGTTC CAGATGAGGA TCGGTTACCT GGGGTAAATT CTTTTCAGGA CACCAGCAAG 840
GTTCTGGCTG TCTCACGTGT AATGGTGGCC TGGCAACCAA TCGGCATATC AATGGGAATC 900
TACGATATGT GCCACAGGTA TCTGAAGGAG AGGAAACAGT TTGGAGCACC GTTGGCTGCT 960
TTCCAGTTAA ACCAACAGAA GCTTGTGCAG ATGCTGGGTA ACGTTCAAGC GATGTTTCTA 1020
ATGGGTTGGC GTCTCTGCAA GCTGTATGAG ACGGGTCAGA TGACTCCAGG TCAAGCCAGT 1080
TTAGGAAAGG CATGGATTTC ATCAAAAGCG AGAGAAACTG CTTCGCTAGG TCGGGAATTA 1140
CTTGGTGGGA ATGGAATTCT AGCAGATTTT CTGGTAGCAA AGGCTTTCTG TGACCTTGAA 1200
CCCATTTATA CATACGAAGG GACTTATGAT ATAAACACCT TAGTAACAGG GAGGGAAGTA 1260
ACGGGTATTG CGAGTTTCAA ACCGGCTACA CGTAGCCGTC TCTAAGTTAA AAGGTTGTCC 1320
ATTGTTTGTT GTTGTCTGTT GGTTAGTATT GATATTGTGA TGTGGGGTTT ACATCTACAA 1380
ATGTGCCAAA TAATCTGACC CAAAAAGATT TAGGGTCCAA TGGGGGAATA ATATCGTTGT 1440
TGCAAAACTT AATCCAGTTA TTGCTTCTTG CAATATGGAT TCGTCTTGTG GCTTGT 1496

## Description

The present invention relates to plant genes encoding enzymes corresponding to members of the ACDH-family in animals. Said genes include genes encoding isovaleryl-CoA-dehydrogenase (IVD) and glutaryl-CoA-dehydrogenase (GCDH). The present invention also relates to nucleic acid sequences which are complementary to said genes or parts thereof, for example antisense RNA or DNA sequences or ribozymes, which are capable of lowering or inhibiting the synthesis of proteins of the ACDH-family (Acyl-CoA-dehydrogenase-family) in plants. The present invention, furthermore, relates to expression vectors comprising inter alia said genes or the nucleic acid sequences which are complementary to said genes.

Moreover, the present invention relates to methods for enhancing the content or altering the composition of fatty acids and/or amino acids and compounds containing these such as any lipids like oils, waxes and fats or storage proteins in a plant cell or plant or for inhibiting or postponing germination in a seed using the above nucleic acid sequences. Furthermore the present invention relates to the use of any sequence of the β-oxidative pathways in plants for the above aim. Finally, the present invention relates to (i) transgenic seeds, plant cells or plants which show an enhanced content or altered composition of fatty acids and/or amino acids and (ii) seeds, which exhibit inhibited or postponed germination. Said plant material can be obtained by introducing the above nucleic acid sequences.

The oils and other lipids synthesized in plants are of great economical interest, oil seed production contributing a major part of world-wide agricultural economy. Plant oils are not only important contributions to human and animal diet, but can also be used in different industrial non-food technologies such as lubricants, surfactants and plasticizers. Manipulation of the lipid content by gene technology is far advanced in specific alterations of the biosynthetic pathways. Many of the enzymes in lipid synthesis have been identified in plants and their genes have to a large extent already been successfully used to alter composition and structure of the lipid content in oil seed crops. However, much less is known about the other end of lipid metabolism, i. e. the degradation of lipids and fatty acids and also storage proteins, e. g., during the mobilization of stored energy reserves in seeds. While in animals parallel fatty acid degradation pathways have been identified in mitochondria and in peroxisomes, lipid metabolism in plants has been exclusively attributed to the peroxisomal (glyoxysomal) pathway. However, scattered biochemical data suggest an additional parallel pathway also in plant mitochondria (see, e.g., Miernyk et al., Plant Physiol. 95 (1991), 564-569, Dieuaide et a., Biochem. J. 296 (1993), 199-207).

The possibility to manipulate the metabolism of fatty acid degradation or lipid degradation should open novel avenues to enhance the bulk storage of fatty acids or compounds containing these such as lipids for example in oil seeds by lowering the activity of enzymes of the ACDH-family or other enzymes of the β-oxidative pathways. The above holds analogously true for the degradation pathway of branched chain amino acids and lysine which is partially accomplished by enzymes of the ACDH-family of enzymes, such as the IVD and GCDH. Again, manipulation of the respective degradative enzyme can be used to enhance protein deposition in seeds and other storage organs. In addition, by lowering or inhibiting the activity of said enzymes, the germination of seeds can be delayed or inhibited. However, for doing so, a detailed knowledge of (i) the enzymes involved in said pathway in plants and (ii) the genes encoding said enzymes is required.

Therefore, the technical problem underlying the present invention is the identification of genes in plants which encode enzymes of the ACDH-family of enzymes in plants, thus allowing to develop tools for the above discussed purposes.

The solution of the above technical problem was achieved by providing the embodiments characterized in the claims.

In animal mitochondria and peroxisomes, the first step of fatty acid β-oxidation is catalyzed by different enzymes. While in peroxisomes the initial step is mediated by the Acyl-CoA-oxidases, the analogous reaction in mitochondria is carried out by Acyl-CoA-dehydrogenases (ACDH), these latter being evolutionary related members of a single gene family. These related enzymes differ in their substrate specificities, converting short, medium, long and very long chain CoA-thioesters to the respective 2,3-enoyl-CoA compounds. Several members of this enzyme family are involved in the catabolism of branched chain amino acids such as leucine and in the lysine oxidative pathway. In plants the degradation pathways of fatty acids, branched chain amino acids and lysine have remained largely unclear. While biochemical evidence generally agrees on β-oxidative activities in glyoxysomes, potential parallel pathways in mitochondria are still debated. In animals distinct families of enzymes are specific to each organelle for the initial step of β-oxidative metabolism, the acyl-CoA-oxidases in peroxisomes and the acyl-CoA-dehydrogenases (ACDH) in mitochondria. The first members of this latter gene family in plants, the IVD and GCDH from *Arabidopsis thaliana*, could now be identified. Surprisingly, the plant gene most similar to the GCDH of the oxidative degradation pathway of lysine has aquired a glyoxysomal target sequence and is no longer imported into mitochondria. A different member of this phylogenetically related gene family, the isovaleryl-CoA-dehydrogenase (IVD), is also in plants localized in mitochondria, showing that this organelle has retained at least part of the β-oxidation pathway of branched chain amino acid degradation. The first step of dehydrating the ester of a fatty acid and coenzyme A is in mammalian mitochondria exclusively catalyzed by members of this gene family, each enzyme recognizing different chain lengths and configurations of the fatty acid moiety. In animals specific enzymes and their respective genes have been described for unbranched and saturated very long, long, medium and short chain acyl-CoA esters respectively, while in plants only the classic multifunctional enzyme pathway of peroxisomes has been dissected so far (Kindl, Biochimie 75 (1993), 225-230).

The availability of the first members of this gene family accesses detailed investigation of leucine, lysine and fatty acid degradation pathways in plants. Clearly, the identification of the IVD and putative GCDH encoding genes now in plants allows to isolate other genes for enzymes of the β-oxidation in plants, those which are members of this same ACDH-family of enzymes. The surprisingly high sequence conservation between the animal and plant ACDH enzymes found in the present invention facilitates this isolation. The more than 60 % identical amino acids between the human/rat and the plant IVD amino acid sequences (Table 1) is in the range of the very conserved respiratory chain subunits encoded in the mitochondrial genomes, such as the cytochrome-c-oxidase subunits, which have just over 50 % of the amino acids in common between plants and animals. Thus, DNA sequences encoding enzymes of the ACDH family e.g. enzymes involved in the β-oxidation pathway in plants can, for example, be isolated by using the DNA sequence of the mammalian counterpart as a probe for hybridization. Alternatively or additionally the here identified plant IVD and GCDH sequences can be used as probes. Suitable methods are well known to the person skilled in the art. These DNA sequences include those for the related members of the ACDH enzyme family in plants with divergent enzymatic specificities in branched chain amino acid and/or fatty acid catabolism, including specificities for branched, unbranched, unsaturated and saturated very long, long, medium and short chain acyl-CoA esters and related branched chain amino acids and the lysine degradative pathway, respectively.

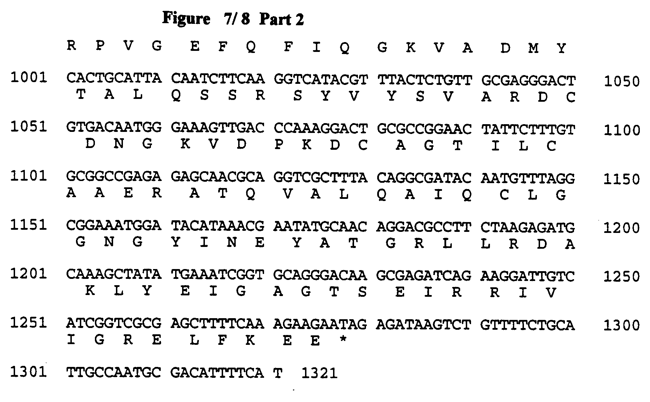

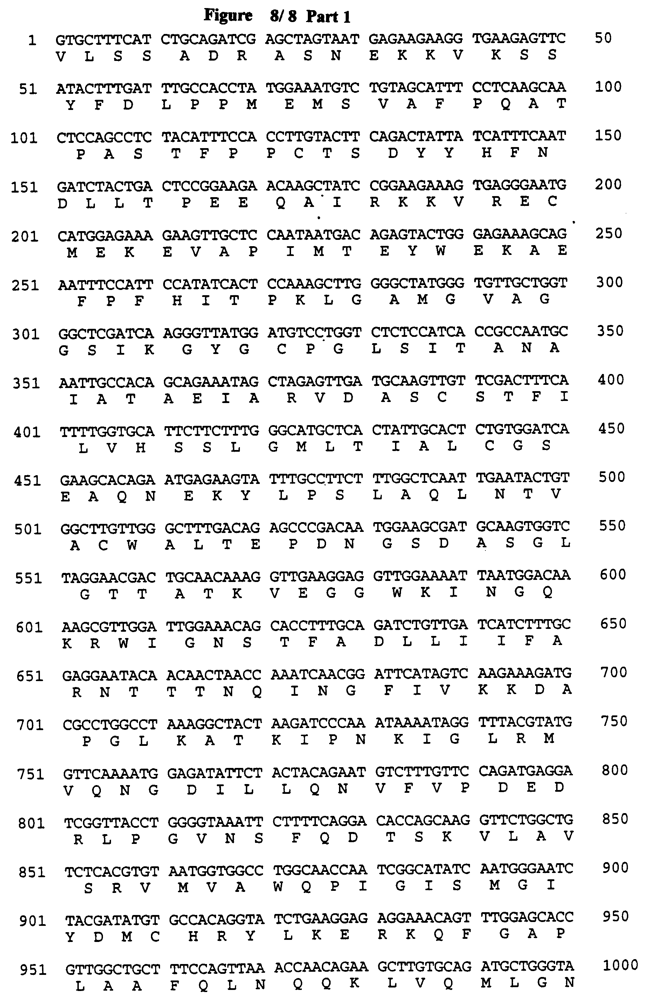

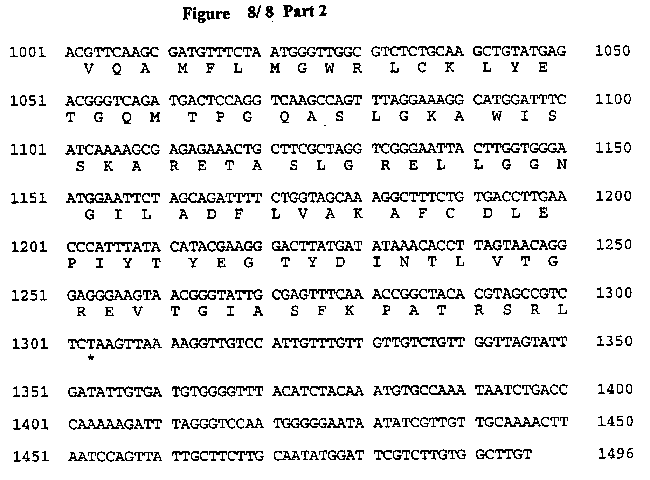

Accordingly, the present invention relates to genes encoding enzymes of the ACDH-family in plants. Said genes also comprise genes encoding isovaleryl-CoA-dehydrogenase (IVD) comprising the nucleic acid sequence shown in Figure 7 or glutaryl-CoA-dehydrogenase (GCDH) comprising the nucleic acid sequence shown in Figure 8. The clones IVD and GCDH are deposited with the DSMZ under accession numbers DSM 11650 and 11651 respectively.

In a preferred embodiment, the gene encodes IVD having the amino acid sequence shown in Figure 7 or GCDH having the amino acid sequence shown in Figure 8.

It has been found that a member of the mammalian mitochondrial ACDH family, the IVD, is located in mitochondria of plants. The here identified IVD in plants is clearly classified as a mitochondrial protein similar to its homologue in animals. Firstly, the pea IVD protein was isolated from purified mitochondria. Secondly, the cleaved presequence of this protein shows all characteristics of a mitochondrial amphiphilic signal peptide as predicted by the computer analyses, while all the peroxisomal signal structures such as the C-terminal SKL are completely lacking. Thirdly, fractionation of cellular structures shows the IVD polypeptide to copurify with mitochondria and not with the peroxisomal fraction. The IVD is thus a mitochondrial enzyme in plants as well as in animals.

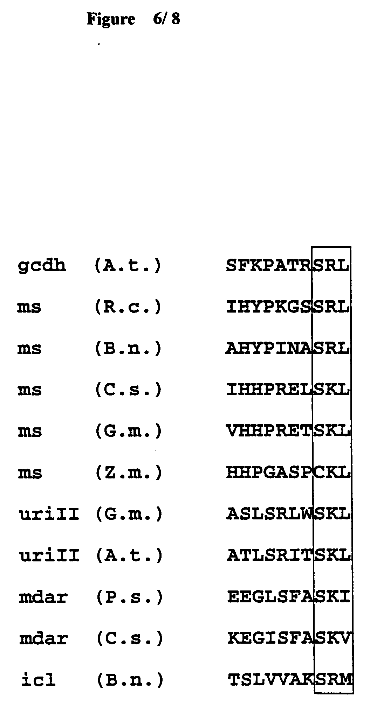

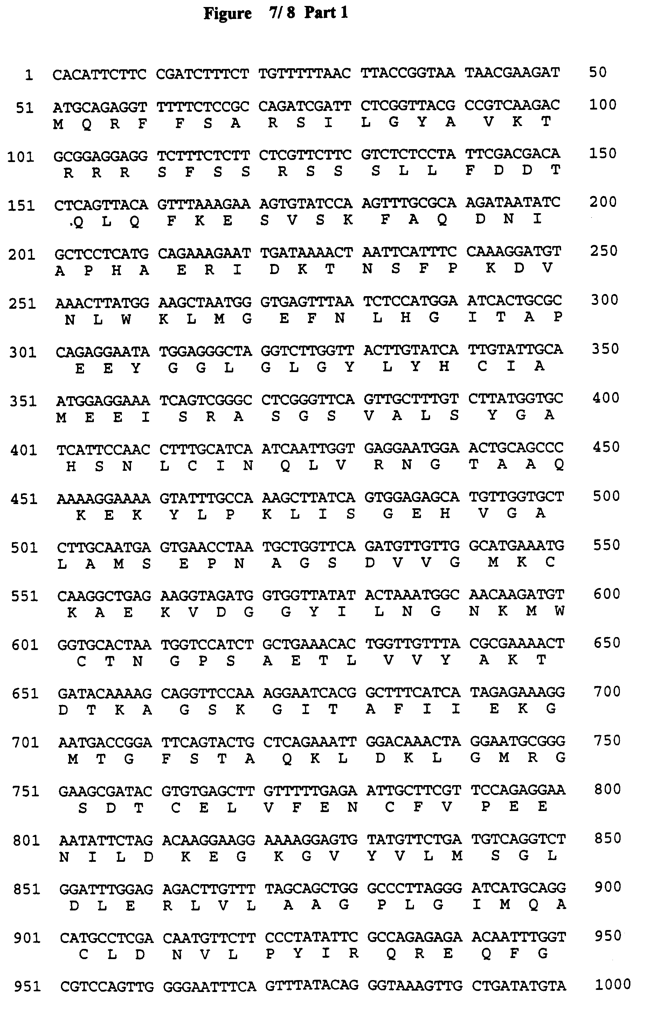

In contrast to the IVD, another member of this enzyme family, the putative GCDH, is directed to peroxisomes in plants. This latter protein is in this text simply referred to as GCDH. This usually (i.e. in animals) mitochondrial protein, a plant acyl-CoA-dehydrogenase (ACDH) with highest similarity to mammalian GCDH proteins, appears to be targetted to peroxisomes. Whereas the IVD in the leucine catabolism remains located in the mitochondrion of plants, this putative GCDH involved in the lysine catabolism has been shifted to a peroxisomal location. All predictions for the amino terminal sequence region in various extensions by the prediction server (PSORT WWW server) disfavour a mitochondrial import signal. The C-terminal region of the predicted protein on the other hand matches with classic SRL import signals for peroxisomes (Fig. 6).

The present invention also relates to nucleic acid sequences which are related to the nucleic acid sequences shown in Figures 7 and 8. These sequences include, for example, sequences which differ from the DNA sequences shown in Figures 7 and 8 due to the degeneracy of the genetic code. It will be understood that the person skilled in the art provided with the sequences shown in Figures 7 and 8 would be in a position to use these DNA sequences, a fragment thereof, or a corresponding oligonucleotide in order to isolate related DNA Sequences from genomic or cDNA libaries of any plant.

All the above DNA sequences are within the scope of the present invention. Thus, the present invention also relates to plant DNA sequences which hybridize to the DNA sequence of Figures 7 or 8 or sequences which differ from said sequences due to the degeneracy of the genetic code under conventional hybridization conditions and encode a polypeptide having the biological properties of IVD or GCDH. In this context, the term 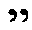 conventional hybridization conditions" refers to hybridization conditions under which the Tₘ value is between Tₘ 35°C and Tₘ 60°C . Preferably, the term 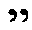 conventional hybridization conditions" refers to relaxed hybridization conditions (buffer: 2 x SSC, 35°C) and, more preferably, to stringent hybridization conditions (buffer: 0.1 x SSC, 60°C).

In a further preferred embodiment, the present invention relates to plant DNA sequences which represent a fragment, allelic variation or other variation of the above DNA sequences, whether said variation results in changes of the protein sequence or not, which still encode a protein having the biological properties of IVD or GCDH. The person skilled in the art can easily determine whether proteins encoded by such DNA sequences still have the biological properties of IVD or GCDH by using routine assays, for example, the assays described below.

In a still further preferred embodiment, the above DNA sequences are genomic DNA sequences or cDNA sequences.

By lowering or inhibiting the expression of genes comprising the above DNA sequences the synthesis of the proteins encoded by said genes can be reduced or eliminated. Thus, in a further embodiment, the present invention relates to nucleotide sequences, i.e. RNA or DNA sequences, which are complementary to the coding strand of the above-mentioned DNA sequences or a part thereof, preferably said nucleotide sequences are complementary to the coding region of the mRNA. These complementary nucleotide sequences, also referred to as 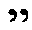 antisense" RNA, DNA, or ribozymes, are known to be capable of lowering or inhibiting the synthesis of the protein encoded by the relevant gene. Said complementary sequences are useful for repression of the gene(s) encoding enzymes of the ACDH family such as those involved in the β-oxidative pathway in plants and the IVD and GCDH. Said sequences and complementary sequences also include those of any other enzymes of the β-oxidative pathways in mitochondria and/or peroxisomes/glyoxisomes to use for the lowering or inhibiting the synthesis of the protein encoded by the relevant gene. A person skilled in the art will find sequences for genes of the β-oxidative pathway(s) in plants which are not members of the ACDH-family, such as the enoyl-CoA-hydratase and the hydroxylacyl-CoA-dehydrogenase, by probing with the respective animal counterparts either by classic experimental hybridisation or by electronic hybridisation to the database entries e.g. in the EST databases using algorithms such as blast search routines. Several of the enzymes and some of the respective nucleic acid sequences of the (in animals) glyoxisomal β-oxidative pathway including the acyl-CoA-synthetase (e.g. Fulda et al., Plant Mol. Biol. 33 (1997) 911-922), 3-ketoacyl-CoA-thiolase (e.g. Olesen and Brandt, Plant Physiol. 110 (1996) 714) or acyl-CoA-oxidase (e.g. Grossi et al., Plant Science 105 (1995) 71-80; Do and Huang, Plant Physiol. (1997) in press, accession no.U66299 in the EMBL database) are available in the databases for use by the above method to lower or increase the synthesis of the respective protein or the expression of the gene.

The term 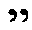 lowering the synthesis of the protein" or 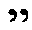 lowering the expression of the gene", as used herein, refers to repression of gene expression to such an extent, that an increase of the content of fatty acids or amino acids and/or compounds containing these can be observed, for example by using the assays described below.

The person skilled in the art provided with the DNA sequences mentioned above will be in position to produce and utilize corresponding antisense RNA or DNA sequences. Suitable approaches are, for example, disclosed in EP-B1 0 223 399 or EP-A1 0 458 367.

Ribozymes which are composed of a single RNA chain are RNA enzymes, i.e. catalytic RNAs, which can intermolecularly cleave a target RNA, for example the mRNA transcribed from the IVD or GCDH genes. The two main requirements for ribozymes are the catalytic domain and region which are complementary to the target RNA and which allow them to bind to its substrate which is a prerequisite for cleavage It is now possible to construct ribozymes which are able to cleave the target RNA at a specific site by following the strategies described in the literatur (see, e.g., Tanner et al., in: Antisense Research and Applications, CRC Press, Inc. (1993), 415-426).

Furthermore, the present invention relates to a vector containing the above genes and DNA sequences, preferably an expression vector comprising a promoter and the genes or DNA sequences of the present invention in operable linkage thereto. In order to ensure the expression of the inserted sequences, any promoter can be used in the expression vector which is functional in plant cells. The expression of said sequences can, in principle, occur in any tissue and at any time. Preferably, however, the expression occurs in tissues in which the lowering, inhibiting or increasing of the expression of genes involved in the synthesis of enzymes of the ACDH family such as IVD and GCDH or other enzymes of the β-oxidative pathway is desirable. Such tissues are for example seeds and any other storage tissues in plants. Thus, preferably, promoters are used in the present invention which allow to induce the specific expression of the inserted DNA sequence in a desired tissue and/or at a desired stage of development. More preferably, said promoters allow to ensure specific expression in seeds, thus allowing to reduce the β-oxidative and/or the lysine and leucine catabolizing activity in growing or germinating seeds. Such promoters are, for example, promoters active during seed maturation and ripening in one or more of the various crop plant species. Furthermore included are promoters which are active in the endosperm or in the cotyledons of seeds and include promoters such as the phaseolin-promoter of *Phaseoulus vulgaris*, the USP-promoter of *Vicia faba* or the HMG-promoter from wheat and mosaic promoters such as the 35S partial promoter or the legumin promoter with specificity boxes from other plant promoters including particularly seed specific elements (e.g. Wobus et al., J. Plant Physiol. 145 (1995) 592-599 and references therein).

In a further preferred embodiment, the above DNA sequences are not only linked to the promoter, but additionally with DNA sequences which allow to increase efficiency of transcription, for example, enhancer elements, and/or 5'- or 3-non-coding sequences which ensure proper termination of trascription and facilitate efficient translation.

The expression vector of the present invention may also suitably comprise a reporter gene, e.g., the uidA gene encoding *E.coli* β-glucuronidase which serves as an indicator of successful transformation. Cells expressing this gene when provided with the substrate (X-Gluc) for the expressed enzyme are capable of converting this substrate into a detectable product (Jefferson et al., EMBO J. 6 (1987), 3901-3907).

The expression vector, preferably, may also comprise a plant selectable marker gene which may, for example be one encoding an antibiotic resistance, e.g. kanamycin resistance (Bevan et al, Nature 304 (1983), 184-187; Jefferson et al., EMBO J. 6 (1987), 3901-3907). Examples of suitable expression vectors are described in standard references (e.g., Celvin et al., Plant Molecular Biology Manual, (1988) Kluwer Academic Publishers Dordrecht, Boston, New York, Supplements and Updates; Jones, ed. Plant Gene Transfer Protocols, (1995) Humana, Totowa, New Jersey).

The above expression vectors are, in addition, useful to enhance the expression of a desired gene encoding an enzyme of the ACDH family such as the IVD or GCDH or any other enzyme active in the β-oxidative pathway(s). In this case said vector carries as an insert such a gene encoding an enzyme as above in plants, for example a gene having the nucleotide sequence displayed in Figures 7 and 8 or the related sequences described above.

A preferred expression vector is pBI (Bevan et al., Nature 304 (1983), 184-187; Jefferson et al., EMBO J. 6 (1987), 3901-3907).

In a further preferred embodiment, the above sequences useful to lower or inhibit expression of a gene encoding an ACDH enzyme involved in the leucine or lysine amino acid catabolism or in the β-oxidative pathway are stably integrated into the genome of the desired plant cell using vectors such as BinAR (Höfgen and Willmitzer, Plant Sci (1990), 221-230).

Additionally, the present invention relates to a method for enhancing the content of fatty acids and/or the above amino acids or compounds containing these in a seed, plant cell or plant comprising introducing the antisense RNA or DNA sequence, the ribozyme or the expression vector discussed above thus, specifically lowering or inhibiting the expression of the gene(s) encoding IVD, GCDH and/or other enzymes of the ACDH family and/or any protein of the β-oxidation pathway(s) in said seed, plant cell or plant.

As used herein, the term 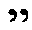 compounds containing fatty acids" refers to all chemical compounds in plants containing fatty acids such as the combinations with glycerine in e.g. oils, fats, waxes and any other lipids. The term 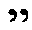 compounds containing amino acids" refers to chemical compounds containing the amino acids leucine and/or lysine including all proteins, particularly storage proteins in whatever tissue appropriate. This furthermore includes the enhanced or altered production of genetically modified proteins in plants, such as elevated lysine proteins in maize kernels, the accumulation of which will be stimulated by enhanced levels of lysine pools through lowered catabolic levels of the GCDH by the various approaches outlined above.

Suitable methods for transforming plants are well known to the person skilled in the art and involve Agrobacterium-based methods, electroporation, microinjection, microprojectile bombardment and vacuuminfiltration. Methods to regenerate, grow and amplify plant material of various plant species notably those of commercial interest such as oil-seed plants, e.g. rape, sunflower, soybean using plant cells as protoplasts, leaf discs, whole plants or in any other form (Bechtold et al., G.r.hebd.Seanc.Acad.Sci.Paris 316 (1993), 1194-1199) are described among others in standard references (e.g., Gelvin et al., Plant Molecular Biology Manual, (1988) Kluwer Academic Publishers Dordrecht, Boston, New York, Supplements and Updates; Jones, ed. Plant Gene Transfer Protocols, (1995) Humana, Totowa, New Jersey).

The identification and the evaluation of transformants with respect to the determination of the enhanced fatty acid or amino acid content can also be carried out by standard methods. Transformants are identified, e.g., due to their resistance to kanamycin and the seeds or regenerated plants are assayed with respect to oil- and/or fatty acids and/or amino acids and/or the respective enzymatic activity according to wellknown methods as, e.g., described in Allen and Good, Meth. Enzymol. 23 (1971), 523-547; Chapman and Barber, Meth. Enzymol. 148 (1987), 294-319).

The reduction of expression of the desired gene can also be determined by analysing the mRNA- and/or protein concentration or by using antibodies which specifically bind to the protein. Alternatively, the activity of some enzymes present in the crude plant extracts can be determined (Allen and Good, Meth. Enzymol. 23 (1971), 523-547; Chapman and Barber, Meth. Enzymol. 148 (1987), 294-319; and standard references and citations therein (e.g., Gelvin et al., Plant Molecular Biology Manual, (1988) Kluwer Academic Publishers Dordrecht, Boston, New York, Supplements and Updates; Jones, ed. Plant Gene Transfer Protocols, (1995) Humana, Totowa, New Jersey).

The above method, additionally, can be used in order to alter the composition of fatty acids and/or amino acids or compounds containing these in the desired plant tissue, *e.g*. seeds, by selectively lowering or inhibiting the expression of a gene or genes encoding a particular enzyme of the β-oxidative and/or the amino acid catabolic pathway and/or by selectively enhancing the expression of a gene or genes encoding a different enzyme of the β-oxidative pathway. For example, the percentages of fatty acid with intermediate chain length can be enhanced by antisense or ribozyme suppression of the intermediate chain length acyl-CoA dehydrogenase(s) and/or by overexpressing the respectively other acyl-CoA dehydrogenase(s) with different chain length specificities. The composition and differential accumulation of proteins can be altered by increasing the pool of certain amino acids such as leucine or lysine by suppressing specific degradation by members of the acyl-CoA- dehydrogenase family (ACDH) such as the IVD and GCDH. Furthermore the percentage of lipids as e.g. seed storage molecules can be altered, increased through a diminished pool of available amino acids in the developing seed by enhancing the degradation of amino acids e.g. through overexpression of the respective degradative enzymes such as the IVD or the GCDH in the respective transgenic tissues of plants. In such an approach an overexpressed and thus increased IVD and/or GCDH activity will reduce the available amino acids and thus reduce the amount of storage proteins made. Thus even when unaltered amounts of storage lipids are made, the purity of these compounds will be increased. The analogous assay holds true for the increase in storage proteins through increased turnover of fatty acids. This effect may be increased through parallel lowering the fatty acid degradation by antisense or ribozyme inhibition of the respective ACDH or other β-oxidative enzymes.

In a further embodiment, the present invention relates to a method for inhibiting or postponing germination in seeds comprising introducing the antisense RNA or DNA sequence, the ribozyme or the expression vector discussed above, thus allowing to specifically lower or inhibit the expression of the gene(s) encoding IVD, GCDH and/or any other protein of the ACDH family and/or any other enzyme of the β-oxidation pathway(s) in said seed.

Transformation of the plant cells, identification of transformants, determination of fatty acid content etc. is carried out as described above. Suitable promoters include promoters which are active in the seed activation of storage proteins or fatty acids and in the appropriate plant cell tissues, e.g. the pre- and early-germination phase and the endosperm or in the cotyledons of seeds and include promoters such as the phaseolin-promoter of Phaseolus vulgaris, the USP-promoter of Vicia faba or the HMG-promoter from wheat and mosaic promoters such as the 35S partial promoter or the legumin promoter with specificity boxes from other plant promoters including particularly seed and/or germination specific elements (e.g. Wobus et al., J. Plant Physiol. 145 (1995) 592-599 and references therein). Further examples are described among other loci in standard references (e.g., Gelvin et al., Plant Molecular Biology Manual, (1988) Kluwer Academic Publishers Dordrecht, Boston, New York, Supplements and Updates; Jones, ed. Plant Gene Transfer Protocols, (1995) Humana, Totowa, New Jersey).

In a preferred embodiment, the protein(s) of the ACDH family and of the β-oxidative pathway(s) in plants, the synthesis of which is manipulated, is/are activities of IVD, GCDH and/or any other enzyme of the ACDH family, such as those with β-oxidative specificities for diverse stages of branched, unbranched, unsaturated and saturated bonds and structures in very long, long, medium and short chain acyl-coA esters and furthermore include unrelated enzymes of the β-oxidative pathway(s) in plants such as acyl-CoA-synthetase (e.g. Fulda et al., Plant Mol. Biol. 33 (1997) 911-922), enoyl-CoA-hydratase, hydroxylacyl-CoA-dehydrogenase, 3-ketoacyl-CoA-thiolase (e.g. Olesen and Brandt, Plant Physiol. 110 (1996) 714) or acyl-CoA-oxidase (e.g. Grossi et al., Plant Science 105 (1995) 71-80; Do and Huang, Plant Physiol. (1997) in press, accession no.U66299 in the EMBL database) and isoforms (The references relate to the proteins and isoforms in specific plant species and their respective counterparts in any desirable crop plant can be identified as described above and below). Further proteins active in glyoxisomal fatty acid β-oxidation such as the tetrafunctional protein (Preisig-Müller et al., J. Biol. Chem. 268 (1994) 20475-20481) can be additionally used to alter fatty acid and/or amino acid contents and composition in plant cells, particularly storage compartments. Methodology is essentially carried out as described above. The nucleic acid sequences encoding the respective protein are used to isolate the required nucleic acid sequences in the respective target crop plants in direct hybridisation by standard technology (e.g. Sambrook et al., Molecular Cloning: A Laboratory Manual, Second Edition. (1989) Cold Spring Harbor Laboratory Press, Cold Spring Harbor). Nucleic acid sequences rot yet available in plants are identified in the respective plant species of choice by taking the homologous animal nucleic acid sequences as query sequence in TblastN searches of the databases, particularly the plant EST sequences. The obtained sequence tags are used to isolate the respective plant cDNA clones and other nucleic acid sequences. Alternatively and/or additionally will standard hybridization technology directly amplify the respectively desired nucleic acid sequences in the respective plant species of choice.

In a further preferred embodiment, the present invention relates to seeds and plant cells which are obtainable by the above described methods or which contain the above described expression vector and to transgenic plants containing said cells. Such plants can be prepared by regeneration from said plant cells using the methods described above (e.g. Gelvin et al., Plant Molecular Biology Manual, (1988) Kluwer Academic Publishers Dordrecht, Boston, New York, Supplements and Updates; Jones, ed. Plant Gene Transfer Protocols, (1995) Humana, Totowa, New Jersey). Suitable plants include crop plants such as sunflower, rape, bean, pea, cocoa, maize, wheat and the like.

In a still further preferred embodiment, the present invention relates to the use of the above DNA- or RNA sequences for lowering, inhibiting or increasing the expression of a gene(s) encoding an enzyme(s) of the ACDH family involved in amino acid catabolism or a β-oxidation pathway in plants. Preferably, such antisense RNA or DNA sequences or ribozymes are used, which allow to decrease or inhibit the expression of a gene encoding a protein with the biological activity of IVD or GCDH or respective other gene(s) of this ACDH family or others in a β-oxidation pathway in plants.

Finally, the present invention relates to proteins encoded by the genes of the present invention, e.g., genes having the nucleic acid sequences depicted in Figures 7 or 8 or genes having a related sequence as defined above. Such proteins can be obtained by recombinant production using well known techniques. Preferred expression systems for obtaining said proteins include the His-Tag system (QIAexpressionist and references therein, Quiagen GmbH, Hilden, Germany) or the expression systems such as offered by Stratagene GmbH (Heidelberg, Germany).

### Legends to figures

**Fig. 1. Amino acid alignment identifies the plant mitochondrial IVD.**
   The N-terminal sequence of a protein purified from pea mitochondria and the homologous *Arabidopsis thaliana* cDNA sequence are identified as encoding a mitochondrial IVD. Amino acids identical between the human (Hum), the rat (Rat) and the *Arabidopsis* (Ara) protein sequence are shaded.
**Fig. 2. An antibody against mammalian IVD recognizes the plant IVD.**
   An antibody against the human mitochondrial IVD reacts unspecifically with proteins in the total cellar protein fraction (Lane 1), but decorates specifically a polypeptide of 43 kDa in the mitochondrial protein fraction (Lane 2). This size agrees with the prediction from the nucleotide sequence of the IVD gene.

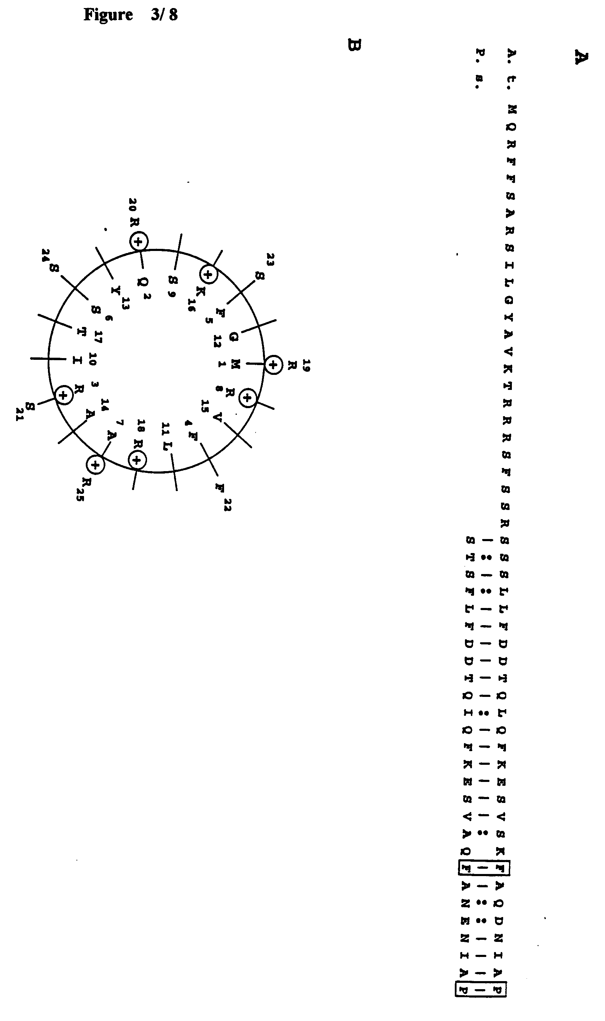

**Fig. 3. The presequence of the IVD shows the typical features of a mitochondrial import signal.**
   (A) The N-terminal sequence of the mature protein isolated from pea mitochondria (P.s.) alignes with the cDNA deduced protein sequence from *Arabidopsis* (A.t.) and defines the cleaved mitochondrial target sequence of 25 amino acids.
   (B) The amphiphilic helix displayed by the presequence of the IVD shows the characteristics of a mitochondrial target signal. Five of seven positive side chains are located to one side and the overall preferred amino acids R, L and S make up almost 50 % of this presequence.

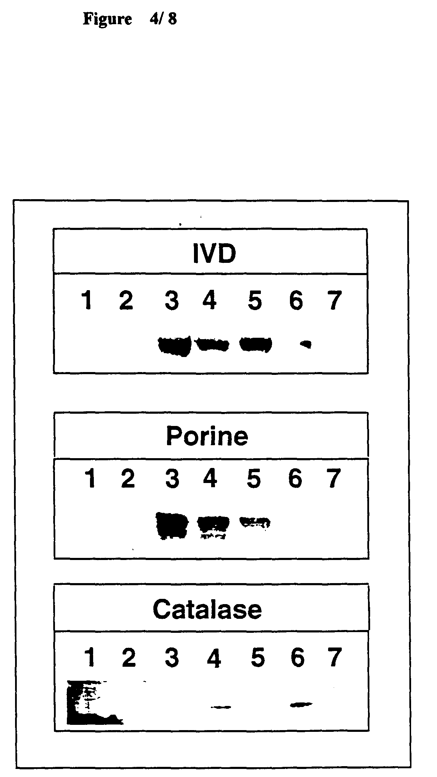

**Fig. 4. The plant IVD protein cofractionates with mitochondrial makers and not with peroxisomal proteins.**
   Pea cellular lysates were fractionated with specific enrichments for mitochondrial and peroxisomal compartments, lanes 1 containing doubled amounts of total cellular proteins, lanes 2 the same quantities of total cellular proteins as lanes 3-7. Lane 3 is a mitochondrial fraction obtained by differential centrifugation and percoll gradient purification. Lanes 4-7 are different sucrose gradient fractions (Gietl et al., Plant Physiol. 113 (1997), 863-871) with lane 4 containing the mitochondrial enrichment at the border of 33/43 % sucrose, lane 5 the interphase at 43/50 %, lane 6 the peroxisomal enrichment at 50/57 % and lane 7 the pellet of the gradient. Quantities loaded were for the IVD blot 180 µg in lane 1 and 90 µg in lanes 2-7, for the porine blot 100 µg in lane 1 and 50 µg in lanes 2-7 and for the catalase blot 150 µg in lane 1 and 75 µg in lanes 2-7.

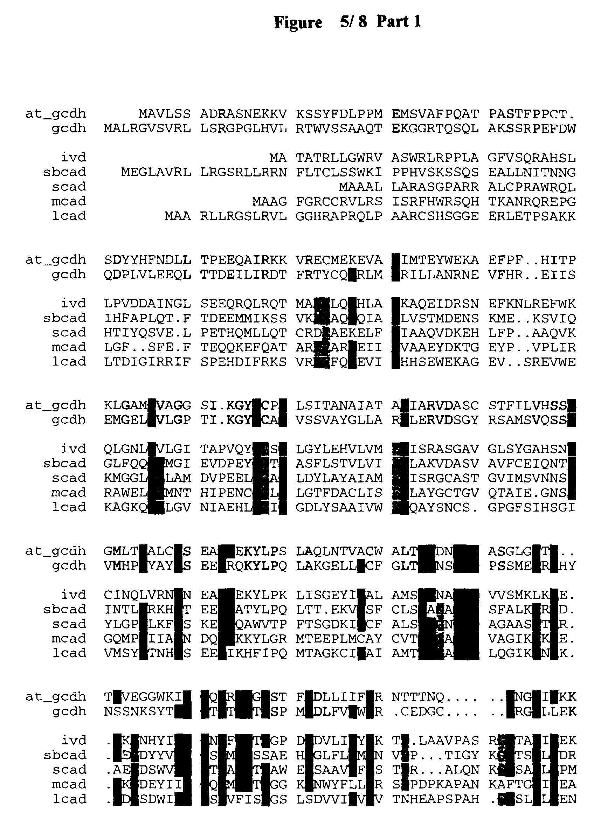

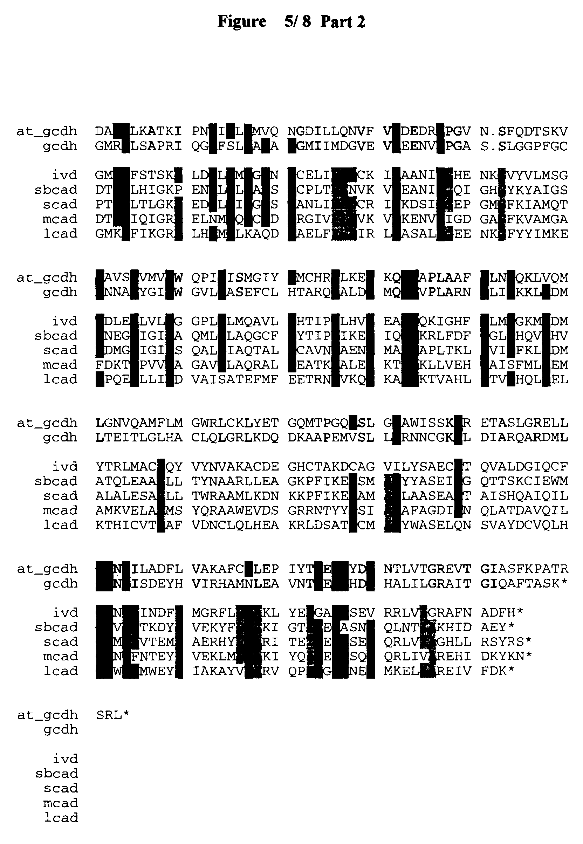

**Fig. 5. Similarities identity the plant glutaryl-CoA-dehydrogenase (GCDH) to be evolutionarily related to the respective animal mitochondrial enzymes.**
   Highest similarity is seen between the putative plant glutaryl-CoA-dehydrogenase (GCDH) in *Arabidopsis* and the mammalian glutaryl-CoA-dehydrogenase (GCDH) enzymes of this family of related enzymes, thus identifying this plant enzyme. Already the IVD and the short branched chain acyl-CoA-dehydrogenase (SBCAD) and the repective enzymes specific for short chain (SCAD), medium chain (MCAD) and long chain (LCAD) fatty acid chains are though clearly related less similar to the GCDH. Light shading identifies amino acids identical between the *Arabidopsis thaliana* GCDH and the human GCDH, intermediate shading highlights four identical out of five between the ivd, sbcad, scad, mcad and lcad alignment block, and a dark background is used where more than six of seven amino acids are identical between all of the different aligned enzyme sequences.
**Fig. 6. The *Arabidopsis thaliana* (A.t.) GCDH C-terminal amino acid identities show the characteristics of a peroxisomal guide sequence.**
   The SRL triplet is clearly a peroxisomal target information, equivalent to the classic peroxisomal C-terminus SKL. One peroxisomal protein such as the malate synthase (ms) can in different plant species have variants of the SKL-triplet, including the SRL in *Ricinus communis* (R.c.) and in *Brassica napus* (B.n.), CKL in *Zea mays* (Z.m.) and SKL in *Cucumis sativa* (C.s.) and *Glycine max* (G.m.). Compared are also uricase II (uriII), the monohydroascorbate reductase with SKI in *Pisum sativum* (P.s.) and SKV in *Cucumis sativa* and the isocitratelyase (icl) with SRM in *Brassica napus*. These similarities strongly suggest the in animals usually mitochondrial enzyme GCDH to be targetted to the peroxisome in plants.
**Fig. 7. Nucleic acid sequence of the cDNA encoding IVD and the deduced amino acid sequence.**
   The sequence given represents the insert of the cDNA clone as deposited with the DSMZ (Accession number DSM 11650). The deduced amino acid sequence is given in the standard single letter code.
**Fig. 8. Nucleic acid sequence of the cDNA encoding GCDH and the deduced amino acid sequence.**
   The sequence given represents the insert of the cDNA clone as deposited with the DSMZ (Accession number DSM 11651). This sequence was determined to be identical to the sequence given in EMBL database accession number U72505, but lacking the first few nucleotides. The deduced amino acid sequence is given in the standard single letter code.

The following examples illustrate the invention

### Example 1: Identification of an IVD cDNA sequence in plants

### General methods:

Gene specific clones were identified in a cDNA library available at the Arabidopsis stock center (Kieber et al., Cell 72 (1993) 427-441). Oligonucleotides A44-1 5'-GAAAGTGTATCCAAGTTTGCGC-3' and A44-2 5'-TTCGTCGGCACTCAGTTACAG-3' were deduced from an Arabidopsis EST clone identified in a blast search with an amino acid sequence from 43 kDa protein isolated from pea mitochondria and were used to isolate an IVD specific clone.
Single plaques were used for in vitro excision of pBluescript II SK+ vectors containing the respective cDNA inserts. Excision procedures were performed as described in the lambda ZAP-II manual (Stratagene). DNA was sequenced using Cy5 AutoRead sequencing kits with Cy5-dATP labelling mix (Pharmacia) and Thermo Sequenase fluorescent label kit (Amersham) following the instructions given by the manufaturers. Sequencing products were analysed on an ALFexpress personal sequencer (Pharmacia). Similarity searches were performed using fasta (Pearson and Lipman, Proc. Natl. Acad. Sci. USA 85 (1988) 2444-2448) and blast (Altschul et al., J. Mol. Biol. 215 (1990) 403-410) algorithms from GCG Wisconsin Package version 9.0-UNIX. Analysis of potential protein sorting was done on a signalpeptide prediction server (PSORT WWW server).

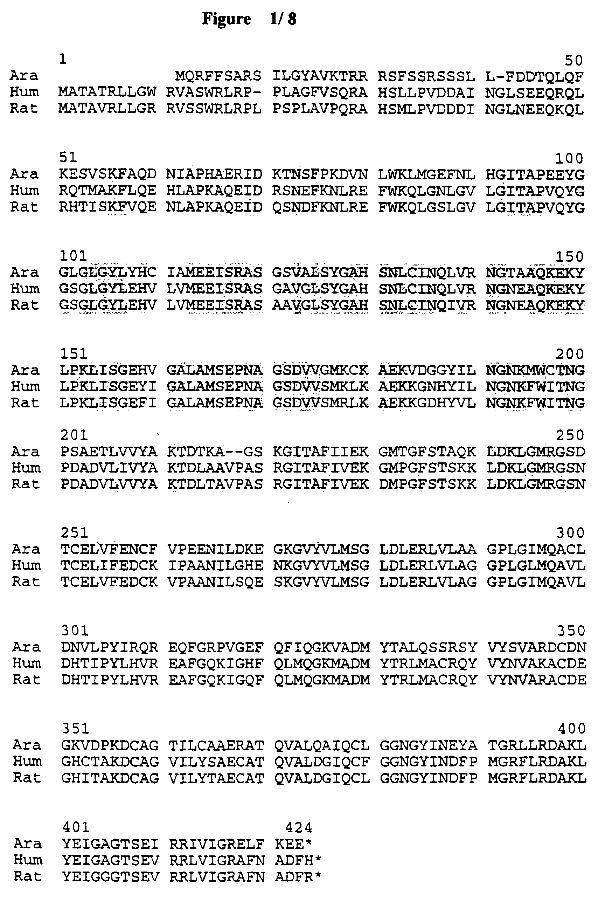

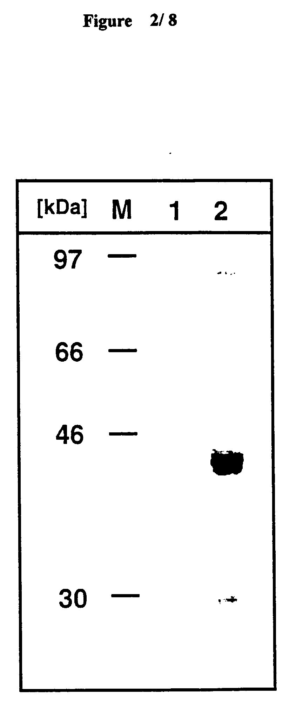

First contact with the ACDH gene family encoding in animals among others enzymes of mitochondrial β-oxidation was a serendipitous observation during the analysis of nucleic acid binding proteins from pea mitochondria. One of the proteins binding to the mitochondrial promoter sequences is isolated as a 43 kDa polypeptide with (apparently specific) affinity to the promoter region as observed in gel shift experiments. The 27 amino acids long N-terminal sequence obtained from this protein was first used to search the databases for related sequences. A matching EST sequence from *Arabidopsis thaliana* was found, and both the N-terminal protein sequence and the identified EST sequence unambiguously score with the ACDH family of genes in similarity searches of the databases. To allow a more stringent identification a full-length clone was isolated from an *Arabidopsis* cDNA library and its complete sequence was determined.
The open reading frame in this clone clearly assigns the encoded protein to the family of acyl-CoA-dehydrogenases comprehensively investigated in mammalia. Detailed alignment furthermore allowed its assignment as an isovaleryl-CoA-dehydrogenase (IVD; Fig. 1 and Table 1). Identity between the plant and mammalian homologues is with more than 60 % of the amino acids extremely high (Table 1). The percentages of identical amino acids between the plant IVD and other members of the ACDH-family of proteins are in the rage of 31-37 %, comparable to the similarities amongst the different mammalian enzymes. Similarities to the less specific bacterial ACDH-enzymes are with just over 40% identical amino acids slightly higher than to the various members of the mammalian enzyme family, but do not reach the IVD identity of more than 60 %. Immunodecoration with an antibody against the mammalian IVD shows that the high similarity is sufficient for a clear crossreaction to the plant homologue (Fig. 2). The western blot furthermore confirms this protein to be expressed in plants. The size of the protein purified from pea mitochondria agrees well with the observed 43 kDa identified in this blot, again confirming the identity of the protein.
The N-terminal sequence of the mature protein purified from pea mitochondria delimits a 25 amino acids long presequence in the *Arabidopsis thaliana* cDNA derived protein sequence (Fig. 2A). This presequence does not contain the N-terminal peroxisomal target sequence PTS2 (RL/I-XXXXX-H/QL) identified in several peroxisomal proteins from plants (Gietl et al., Plant Physiol. 113 (1997) 863-871). Twelve of the 25 amino acids in this presequence are arginine, leucine or serine, a enrichment characteristic for mitochondrial target-sequences (von Heijne et al., Eur. J. Biochem. 180 (1989) 535-545). Indeed, this presequence can be folded into a typical amphiphilic helix, in which most of the positively charged amino acids cluster on one side. Computer predictions of the complete amino acid sequence by PSORT WWW server clearly indicate mitochondrial targetting characteristics, further corroborating the mitochondrial location of the encoded enzyme in plants.
The subcellular location of the IVD protein was experimentally analyzed by western blot analysis of fractionated cells (Fig. 4). Subcellular fractionation and protein analyses were carried out as follows:
A method for the isolation of microbodies from castor bean endosperm (Gietl et al., Plant Physiol. 113 (1997) 863-871) was used to fractionate subcellular compartments from etiolated pea seedlings (Var. Lancet or Progress Nr. 9). 5 days old seedlings were harvested and homogenized in 3 ml per g fresh weight of buffer H containing 50 mM Hepes pH 7.4, 10 mM KCl, 1 mM EDTA, 1 mM DTT. After removing cell debris in a centrifgation at 2,000 x g, a crude organellar pellet was collected from the supernatant by centrifugation at 10,500 x g for 15 min. The organelles were resuspended in 1 ml buffer H containing 0.8 % sucrose and loaded onto a discontinuous gradient composed of 1 ml 10%, 1.5 ml 20%, 2.0 ml 33%, 2.0 ml 43%, 2.0 ml 50% and 2.0 ml 57% sucrose in 10 mM EDTA pH7.4 and 5 mM MgCl₂. After centrifugation at 111,100 x g for 2 h, probes were removed from each interphase and from the pellet and slowly adapted to lower sucrose concentrations by an eightfold dilution with buffer H. Organelles from each fraction were collected by centrifugation at 10,000 x g for 15 min washed in buffer H containing 15 % glycerol and subsequently frozen in liquid nitrogen.

Subcellular fractions were resupended in lysis buffer containing 100 mM Tris-HCl pH 8.0, 0.2 mM EDTA, 1 mM DTT, 1 mM PMSF and 15 % glycerol. Organellar components were lysed by addition of SDS to a final concentration of 2 %. Total cellular protein extracts from pea seedlings were prepared as described (Shewry et al., in Plant Gene Transfer Protocols, ed. Jones, H. (1996) Humana, Totowa, New Jersey, pp. 399-422). After measuring protein concentrations (Biorad protein assay), 30 - 90 µg of protein were separated by SDS-PAGE and blotted onto PVDF membranes (Roth) following standard protocols (Sambrook et al., Molecular Cloning: A Laboratory Manual, Second Edition, (1989) Cold Spring Harbor Press, Cold Spring Harbor, New York). Proteins were selectively detected using respective antibodies and a Vectastain ABC kit (Vector laboratories) according to the suppliers instructions. Separation of mitochondria and peroxisomes was monitored by antibodies against porine and catalase respectively, showing the relative enrichment of the mitochondrial marker protein to parallel the protein abundance of the IVD. This result confirms the mitochondrial location of the IVD and furthermore shows that this enzyme is not targetted to peroxisomes.

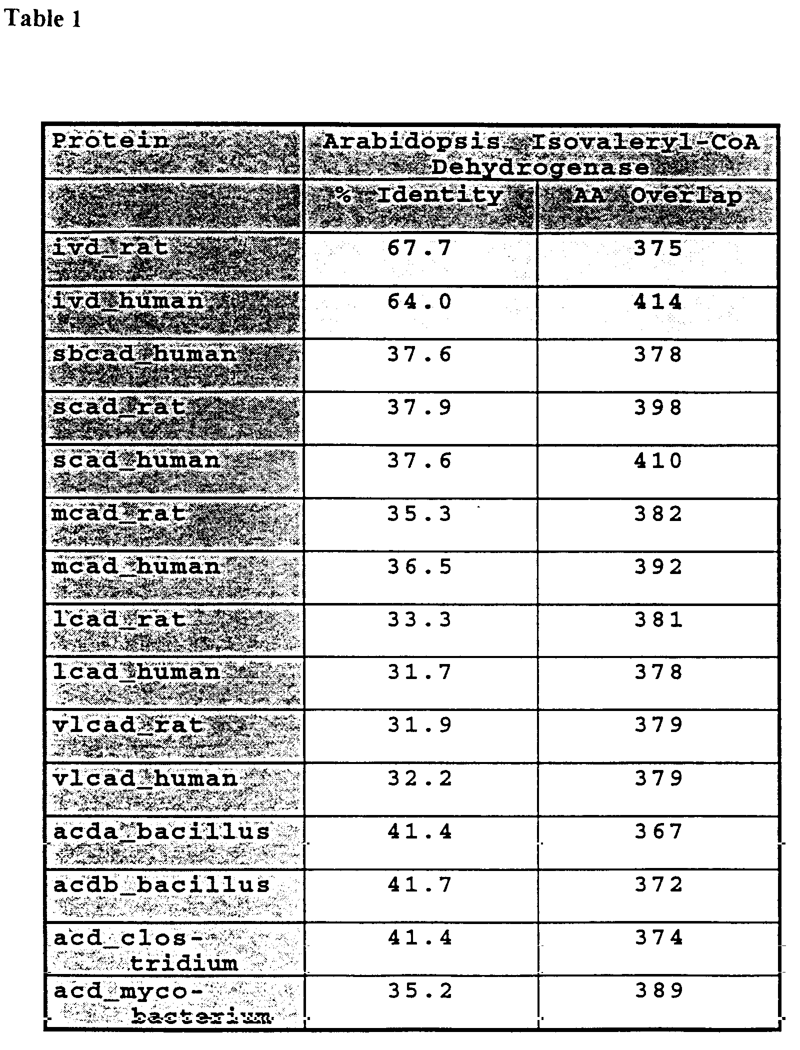

Table 1. Similarities to different enzymes of the mitochondrial ACDH family in animals identify the plant isovaleryl-CoA-dehydrogenase IVD. The 64 and 67.7 % amino acids identical between the *Arabidopsis* deduced protein sequence and the human and rat IVD enzymes are significantly higher than the similarity to other members of the mitochondrial ACDH family. Abbreviations are sbcad, short branched chain acyl-CoA-dehydrogenase; scad, short chain acyl-CoA-dehydrogenase; mcad, medium chain acyl-CoA-dehydrogenase; lcad, long chain acyl-CoA-dehydrogenase; vlcad very long chain acyl-CoA-dehydrogenase and acd, acyl-CoA-dehydrogenase. The different sequences can be found in the databases.

### Example 2: Identification of an ACDH cDNA in plants with high similarity to Glutaryl-CoA-Dehydrogenase (GCDH)

Searching the plant EST-databases with the *Arabidopsis thaliana* IVD and various mammalian ACDH amino acid sequences of this gene family as query sequences identified several cDNA clones in both the rice and the *Arabidopsis thaliana* EST collections. Short, short branched, medium, long and very long chain fatty acid specific ACDH proteins were used a query sequences in the blast searches. Three overlapping cDNA EST sequences were selected for further investigation and derived oligonucleotides were used to isolate three clones from an *Arabidopsis thaliana* cDNA library. These cDNA clones were identified and isolated with oligonucleotides AAD-1 5'-CGTGTAATGGTGGCCTGGCAACC-3' and AAD-2 5'-GGTCACAGAAAGCCTTGGCTACCAG-3' deduced from Arabidopsis EST sequences identified in blast searches using various mammalian ACDH proteins as query sequences. Screening of the cDNA library followed the instructions given in the lambda ZAP-II manual (Stratagene). Cloning, sequence analysis and computer analyses were carried out according to the methods described in Example 1, above.

The complete sequence of the open reading frame in these clones translates into a protein with a clear association to the mitochondrial acyl-CoA-dehydrogenase family from animals (Table 2). Highest similarity is observed to mammalian Glutaryl-CoA-Dehydrogenase (GCDH) with 37.7 % amino acids being identical with the human GCDH. Considerably lower similarities with 26-30% identical amino acids are observed with other ACDH enzymes (Table 2). An analogous comparison between this human GCDH and the other members of the ACDH family shows an almost identical distribution of similarities between 26 and 32 % identical amino acids for the animal mitochondrial ACDH polypeptides (not shown). Both the plant and human GCDH sequences have 25 to 34 % amino acids identical with the bacterial ACDH enzymes. Similarity to peroxisomal acyl-CoA-oxidase enzymes of animals is much lower, reflecting the separate evolutionary origins of the respective genes.
The sequence alignment of this plant acyl dehydrogenase shows the scattered conserved amino acid positions characteristic for the family of ACDH enzymes, but also reveals that some of the ACDH signature amino acids are lacking in both the plant and in the animal GCDH sequences (Fig. 3). Thus, this plant protein was identified as a very probably GCDH enzyme.
Computer analysis of the protein sequence deduced for the plant GCDH surprisingly predicts none of the typical mitochondrial import signals in this region (not shown).
Neither amphiphilic helix nor a predominance of the typical amino acids are found at the N- terminus. Rather than showing mitochondrial or chloroplast signatures this protein is predicted to be targetted to peroxisomes.
The C-terminal region contains a perfect peroxisomal PTSI import signal. The terminal amino acids SRL constitute one possible arrangement in the family of combinations, which are derived from the classic peroxisomal signal triplet SKL (Fig. 4). These features suggest that the GCDH-like protein is destined to the peroxisomal/glyoxysomal compartment in plant cells.

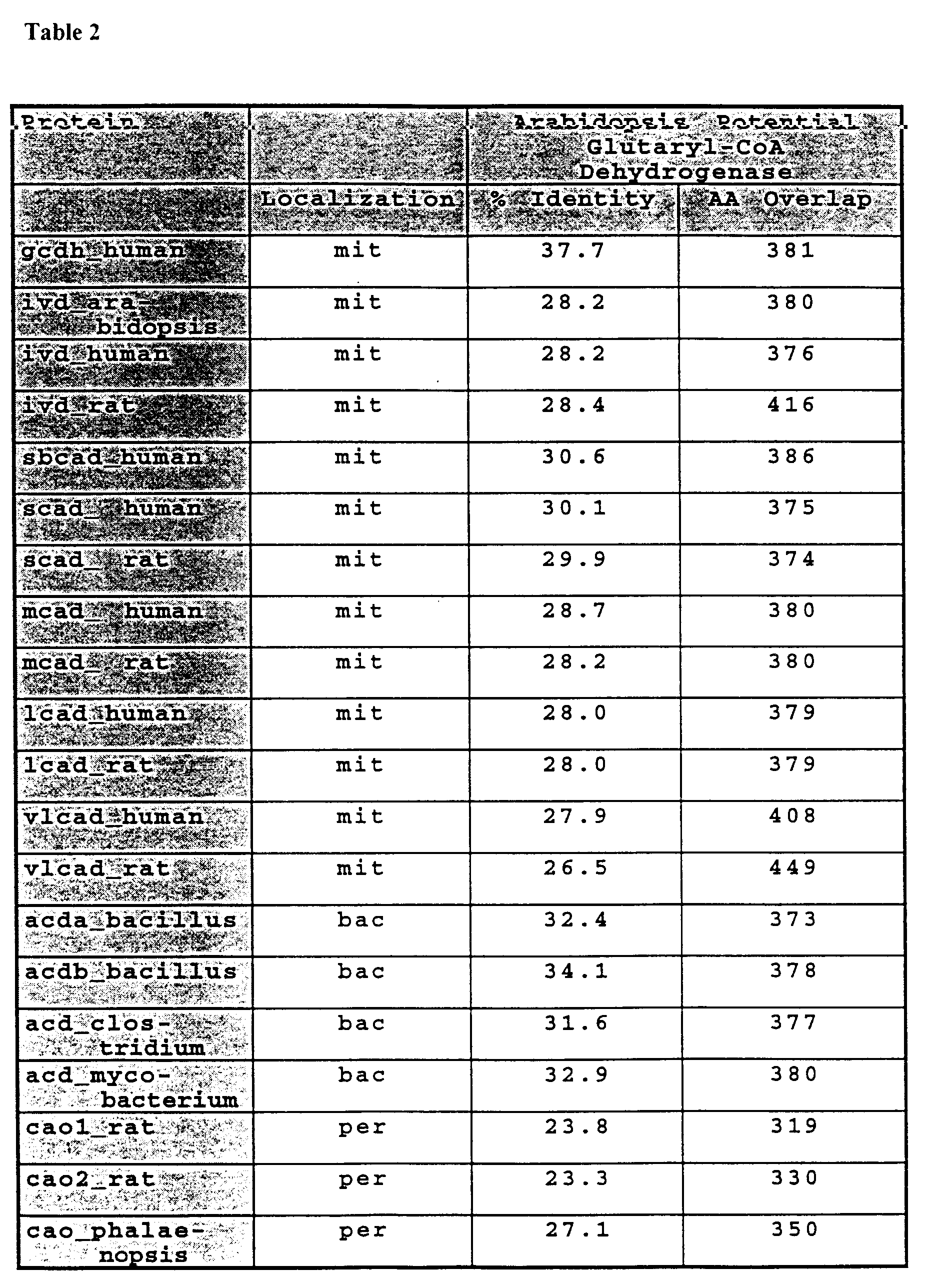

Table 2. Similarities to different enzymes of the mitochondrial ACDH family in animals identify the potential acyl-CoA-dehydrogenase in the flowering plant *Arabidopsis thaliana* as a glutaryl-CoA-dehydrogenase (GCDH). The 37.7 % amino acids identical between the *Arabidopsis* deduced protein sequence and the human GCDH enzyme are significantly higher than the similarity to other members of the mitochondrial ACDH family. Abbreviations are sbcad, short branched chain acyl-CoA-dehydrogenase; scad, short chain acyl-CoA-dehydrogenase; mcad, medium chain acyl-CoA-dehydrogenase; lcad, long chain acyl-CoA-dehydrogenase; vlcad, very long chain acyl-CoA-dehydrogenase; acd, acyl-CoA-dehydrogenase and cao, acyl-CoA-oxidases. The different sequences can be found in the databases. Intracellular locations of the different enzymes are indicated for mitochondria (m), bacteria (b) and peroxisomes (p).

### Example 3: Construction of an expression vector for inhibiting the expression of the gene encoding IVD in Arabidopsis thaliana.

To construct the chimeric IVD antisense gene, a full-length 1.29 kb fragment containing the IVD gene is amplified by PCR with the primers IVDHin 5'-CGGAGCTCTCTTGTTTTTAAC-3' and IVDruck 5'-GCAGGATCCAGACTTATCTC-3' and is cloned in reverse orientation at SacI and BamHI restriction sites into the expression cassette behind the CaMV promoter of pB1121 (Jefferson et al., EMBO J. 6 (1987), 3901-3907) vector where the β-glucuronidase gene has been removed. The resulting plasmid is confirmed to contain the IVD gene in antisense orientation behind the CaMV 35S promoter by sequencing.

### Example 4: Transformation of Arabidopsis thaliana

*Arabidopsis thaliana* was transformed by vacuum infiltration of *Agrobacterium* with the plasmid of Example 3. The procedure followed essentially described protocols cells using methods well known by the person skilled in the art (e.g. Gelvin et al., Plant Molecular Biology Manual, (1988) Kluwer Academic Publishers Dordrecht, Boston, New York, Supplements and Updates; Jones, ed. Plant Gene Transfer Protocols, (1995) Humana, Totowa, New Jersey) and standard technology (e.g. Sambrook et al., Molecular Cloning: A Laboratory Manual, Second Edition. (1989) Cold Spring Harbor Laboratory Press, Cold Spring Harbor). Transgenic antisense plants were regenerated from the seeds selected by their gained Kanamycin resistance. *Arabidopsis thaliana* plants were grown under standard greenhouse conditions and seed pods were harvested upon maturation and seeds were collected to evaluate the content of leucine and the seed proteins, respectively, as described in Example 5, below.

### Example 5: Determination of the IVD activity and the protein content demonstrates successful inhibition of the expression of the leucine degrading enzyme IVD

The activity of the IVD enzyme was determined in lysates of leaves, seed pods and isolated seeds of the above transformant in addition, of an untransformed plant used for comparison following a standard protocol for the enzyme assay (Ikeda et al., J.Biol. Chem. 258 (1983) 1066-1076; Ikeda and Tanaka, J.Biol. Chem. 258 (1983) 1077-1085; Gerhardt, Meth. Enzymol.148 (1987) 516-525).
Protein concentrations were measured by the Biorad protein assay following standard protocols (Sambrook et al., Molecular Cloning: A Laboratory Manual, Second Edition, (1989) Cold Spring Harbor Press, Cold Spring Harbor, New York). It was found that the activity of IVD was significantly reduced in the transformant compared to the control. Moreover, the protein content was increased in the transformant.

### Literature

Allen and Good, Meth. Enzymol. 23 (1971), 523-547.
Altschul et al., J. Mol. Biol. 215 (1990) 403-410.
Bechtold et al., G.r.hebd.Seanc.Acad.Sci.Paris 316 (1993), 1194-1199.
Bevan et al., Nature 304 (1983), 184-187.
Chapman and Barber, Meth. Enzymol. 148 (1987), 294-319.
Dieuaide et a., Biochem. J. 296 (1993), 199-207.
Do and Huang, Plant Physiol. (1997) in press, accession no.U66299 in the EMBL database
Fulda et al., Plant Mol. Biol. 33 (1997) 911-922.
Gelvin et al., Plant Molecular Biology Manual, (1988) Kluwer Academic Publishers
Dordrecht, Boston, New York, Supplements and Updates
Gerhardt, Meth. Enzymol.148 (1987) 516-525.
Gietl et al., Plant Physiol. 113 (1997), 863-871.
Grossi et al., Plant Science 105 (1995) 71-80.
von Heijne et al., Eur. J. Biochem. 180 (1989) 535-545.
Höfgen and Willmitzer, Plant Sci (1990), 221-230.
Ikeda et al., J.Biol. Chem. 258 (1983) 1066-1076.
Ikeda and Tanaka, J.Biol. Chem. 258 (1983) 1077-1085;
Jefferson et al., EMBO J. 6 (1987), 3901-3907.
Jones, ed. Plant Gene Transfer Protocols, (1995) Humana, Totowa, New Jersey Kieber et al., Cell 72 (1993) 427-441.
Kindl, Biochimie 75 (1993), 225-230.
Miernyk et al., Plant Physiol. 95 (1991), 564-569.
Olesen and Brandt, Plant Physiol. 110 (1996) 714.
Pearson and Lipman, Proc. Natl. Acad. Sci. USA 85 (1988) 2444-2448.
Preisig-Müller et al., J. Biol. Chem. 268 (1994) 20475-20481.
Sambrook et al., Molecular Cloning: A Laboratory Manual, Second Edition, (1989) Cold Spring Harbor Press, Cold Spring Harbor, New York.
Shewry et al., in Plant Gene Transfer Protocols, ed. Jones, H. (1996) Humana, Totowa, New Jersey, pp. 399-422.
Tanner et al., in: Antisense Research and Applications, CRC Press, Inc. (1993), 415-426).
Wobus et al., J. Plant Physiol. 145 (1995) 592-599.

## Claims

1. A gene encoding an enzyme of the ACDH family in plants.

2. The gene of claim 1 encoding isovaleryl-CoA-dehydrogenase (IVD) comprising the nucleic acid sequence shown in Figure 7 or glutaryl-CoA-dehydrogenase (GCDH) comprising the nucleic acid sequence shown in Figure 8.

3. The gene of claim 2 encoding IVD having the amino acid sequence shown in Figure 7 or GCDH having the amino acid sequence shown in Figure 8.

4. A plant gene encoding a protein exhibiting the biological properties of IVD or GCDH comprising a DNA sequence:
(a) which differs from the DNA sequence of claim 2 or 3 in codon usage due to the degeneracy of the genetic code;
(b) which hybridises with the DNA sequence of claim 2, 3 or section (a), above; or
(c) which represents a fragment, allelic or other variation of the DNA sequence of claim 2 or 3, whether said variation results in changes in the protein sequence or not.

5. The gene of claim 4, wherein the DNA sequence is a genomic DNA sequence.

6. The gene of claim 4, wherein the DNA sequence is a cDNA sequence.

7. Antisense RNA or DNA sequence characterized in that it is complementary to the gene of anyone of claims 1 to 6 or a part thereof, said sequence being capable of lowering or inhibiting the synthesis of the protein encoded by said gene.

8. Ribozyme characterized in that it is complementary to the gene of any one of claims 1 to 6 and can selectively bind to and cleave a mRNA encoded by said gene, thus lowering or inhibiting the synthesis of the protein encoded by said gene.

9. An expression vector comprising a promoter and the gene or antisense DNA sequence or ribozyme of any one of claims 1 to 8 in operative linkage thereto.

10. The expression vector of claim 9, wherein the promoter is selected from the group consisting of the phaseolin-promoter of *Phaseolus vulgaris*, the USP-promoter *Vicia faba*, the HMG-promoter from wheat, the 35S partial promoter or the legumin-promoter with specificity boxes from other plant promoters or other developing-or seed-specific promoters.

11. The expression vector of claim 9 or 10, which is based on pBI.

12. A method for enhancing the content of fatty acids and/or amino acids or compounds containing these in a seed, plant cell or plant comprising introducing the antisense RNA or DNA sequence of claim 7, the ribozyme of claim 8 or the expression vector of any one of claims 9 to 11, thereby specifically lowering or inhibiting the expression of a gene(s) encoding an enzyme(s) of the ACDH family and/or of the β-oxidation pathway(s) in said seed, plant cell or plant.

13. A method for altering the composition of fatty acids and/or amino acids or compounds containing these in a seed, plant cell or plant comprising introducing the antisense RNA or DNA sequence of claim 7, the ribozyme of claim 8, the gene of claims 1-6 or the expression vector(s) of any one of claims 9 to 11, thereby specifically lowering, inhibiting and/or increasing the expression of a gene(s) encoding an enzyme(s) of the ACDH family and/or of the β-oxidation pathway(s) in said seed, plant cell or plant.

14. A method for inhibiting or postponing germination in seeds comprising introducing the antisense RNA or DNA sequence of claim 7, the ribozyme of claim 8 or the expression vector of claim 9 or 10, thereby specifically lowering or inhibiting the expression of a gene(s) encoding an enzyme(s) of the ACDH family and/or of the β-oxidation pathway(s) in said seed.

15. The method of any one of claims 12 to 14, wherein the enzyme of the mitochondrial β-oxidation pathway is IVD, GCDH, acyl-CoA-synthetase, acyl-CoA-oxidase, enoyl-Co-hydratase, 3-ketoacyl-CoA-thiolase or hydroxylacyl-CoA-dehydrogenase.

16. A seed, plant cell or plant, obtainable by the method of any one of claims 12 to 15.

17. A seed, plant cell or plant, comprising the antisense RNA or DNA sequence of claim 7, the ribozyme of claim 8 and/or the expression vector of any one of claims 9 to 11.

18. The seed, plant cell or plant of claim 16 or 17, which is a monocot or dicot.

19. The seed, plant cell or plant of claim 18, wherein the monocot is maize, wheat or rice and the dicot is soybean, rape, sunflower, bean, pea, thistle, olive, walnut, pumpkin or cocoa.

20. Use of the gene, antisense DNA or RNA sequence or ribozyme of any one of claims 1 to 8 for lowering, inhibiting or increasing the expression of a gene(s) encoding an enzyme(s) of the ACDH family and/or of the β-oxidation pathway(s) in plants.

21. A protein encoded by the gene of any one of claims 1 to 6.
